# EUROPEAN PATENT APPLICATION

(11) **EP 1 348 436 A1**
(43) Date of publication of application: **01.10.2003**
(21) Application number: 02007455.5
(22) Date of filing: 30.03.2002
(51) Int. Cl.: A61K 31/5415, A61K 9/02, A61P 29/00, A61P 19/02, A61K 47/10

(54) **Meloxicam suppositories**

(71) Applicant: Boehringer Ingelheim International GmbH, 55216 Ingelheim am Rhein (DE)
(72) Inventor: Matsui, Mayumi, Ikeda, Osaka (JP); Wada, Koichi, Kawabe-Gun Hyogo 666-0257 (JP); Ohki, Toshimitsu, Ikeda, Osaka 563-0043 (JP)
(74) Representative: Laudien, Dieter, Dr.

(57) **Abstract**

The present invention relates to a suppository essentially consisting of the active ingredient meloxicam or a pharmaceutically acceptable salt thereof and at least one pharmaceutically acceptable excipient.

## Description

### Field of the invention

The present invention relates to a suppository essentially consisting of the active ingredient meloxicam or a pharmaceutically acceptable salt thereof and at least one pharmaceutically acceptable excipient.

### Background of the invention

Meloxicam [2H-1,2-benzothiazine-3-carboxamide, 4-hydroxy-2-methyl-N-(5-methyl-2-thiazolyl)-1,1-dioxide] is a non-steroidal antiinflammatory drug (NSAID) with antiinflammatory, antipyretic and analgetic activity.
Rectal absorption of Meloxicam has been reported (Arzneimittelforschung 47, 3, 253, 1997). The type and nature of a suppository base and a solubilizer affect the stability, release and absorption of a drug.
The formulation of meloxicam suppositories is desribed in J. Pharm. Sci. (Vol.23, 11, June 1999), wherein meloxicam is formulated in different suppository bases e.g. Witepsol H15 in combination with PVP K90. The drug release of this formulation is comparable with commercially available meloxicam suppositories.

### Description of the invention

The problem underlying the present invention is to provide a meloxicam suppository, which shows a fast onset of meloxicam absorption and a high bioavailability of meloxicam.

Thus it has surprisingly been found that the problem underlying the invention is solved by a suppository containing a composition, essentially consisting of meloxicam or a pharmaceutically acceptable salt thereof, and at least one pharmaceutically acceptable excipient, wherein at least one of the excipients is a polyalkylenglycol.

According to the invention the term pharmaceutically acceptable salt stands for a meloxicam salt of an organic or inorganic base, as for example the meglumin, sodium, potassium or ammonium salt.
The Polyalkylenglycols are as a rule Polyethylenglycols, Polypropylenglycols, or mixtures thereof, preferably Polyethylenglycols.
The Polyethylenglycol component typically has an average molecular weight from about 200 to about 6000. Commercially available Polyethylenglycol materials, for example from Nippon Yushi Co.,Ltd., include e.g. PEG 400, PEG 800, PEG 1000, PEG 2000, PEG 3000, PEG 4000 and PEG 6000.

In a preferred embodiment the present invention relates to a suppository, wherein the suppository is a soft gelatine capsule suitable for rectal administration.

In a further preferred embodiment the present invention relates to a suppository wherein the composition comprises the meloxicam meglumin salt.

In a further preferred embodiment the present invention relates to a suppository wherein the composition comprises the free meloxicam base.

In a particularly preferred embodiment the present invention relates to a suppository, wherein the polyalkylenglycol is selected from the group consisting of polyethylenglycol 400, polyethylenglycol 4000 and polyethylenglycol 6000.

In a further particularly preferred embodiment the present invention relates to a suppository which contains 0.1 % by weight or more, preferably 0.1 % to 5 %, more preferably about 0.3% of a solubilizer or a mixture of solubilizers of the overall weight of said composition.
According to the invention solubilizers are preferably selected from the group consisting of sodium hydroxide, potasium hydroxide, sodium bicarbonate, sodium citrate, and Meglumin.

In a further particularly preferred embodiment the present invention relates to a suppository, wherein the composition contains 1 to 20 µg, preferably 3 to 15 µg, more preferably about 5 to 10 µg, of the free meloxicam base per mg of said composition.

In another particularly preferred embodiment the present invention relates to a suppository which contains at least 0.2 mg, preferably 0.2 to 5 mg, more preferably about 1 mg, of the solubilizer or mixture of solubilizers per mg of the free meloxicam base.

More particularly preferred according to the present invention is a suppository, which contains a composition consisting of
a) 1 to 20 mg, preferably 3 to 15 mg, more preferably about 5 to 10 mg, of meloxicam,
b) 500 to 1500 mg, preferably 800 to 1200 mg, more preferably about 1000 mg, of one or more polyalkylenglycols selected from the group consisting of polyethylenglycol 400, polyethylenglycol 4000 and polyethylenglycol 6000, and optionally
c) one or two additional excipients, e.g. a solubilizer, preferably meglumine or a stabilizer.

As a rule according to the invention a method for preparation of said suppository comprises the steps of
a) intimately mixing of meloxicam or a pharmaceutically acceptable salt thereof with the melted solubilizer, and
b) encapsulating or molding of the composition obtained in step a) by a conventional method, for example described in "Suppositories -From Dosage Forms to Clinical Applications-", Nanzando co., Ltd. 1985.

The suppository of the present invention is used for the preparation of a medicament with enhanced bioavailability for the treatment or prevention of polyarthritis, rheumatoid arthritis or inflammation deseases.

The invention will be further illustrated by the examples of suppository compositions given in Table 1. The invention should not be limited to these examples. According to the present invention the compositions B & C are manufactured to suppositories by conventional methods, which are described for instance in "Suppositories -From Dosage Forms to Clinical Applications-", Nanzando co., Ltd. 1985.

A suitable method of manufacturing a composition according to the invention is e.g. to dispense each component of the composition in the suppository base, e.g. a polyethylenglycol. Subsequently all components are mixed in a suitable vessel, such as a stainless steel beaker, at room temperature. The mixture is stirred for about 0.5 hours, until a clear solution is obtained.

**Table 1.**

| Compositions of meloxicam for suppositories Values are given in [mg]. | | | |
|---|---|---|---|
| Formulation | A | B | C |
| Dosage form | Rectal capsule | Conventional suppository | Conventional suppository |
| Meloxicam | 5.0 | 5.0 | 5.0 |
| Meglumine | 2.8 | - | - |
| Polyethyleneglycol 400 | 992.2 | - | 250 |
| Polyethyleneglycol 4000 | - | 905 | 655 |
| Polyethyleneglycol 6000 | - | 90 | 90 |
| Total weight | 1000 | 1000 | 1000 |

Bioavailability tests have been conducted with suppository formulations according to the present invention and with a commercially available meloxicam formulation. The composition of the tested Meloxicam suppository formulation is based on *fats such as Suppocire BP.* The plasma concentration test is conducted using beagle dogs. Six to nine suppositories were tested for each batch.

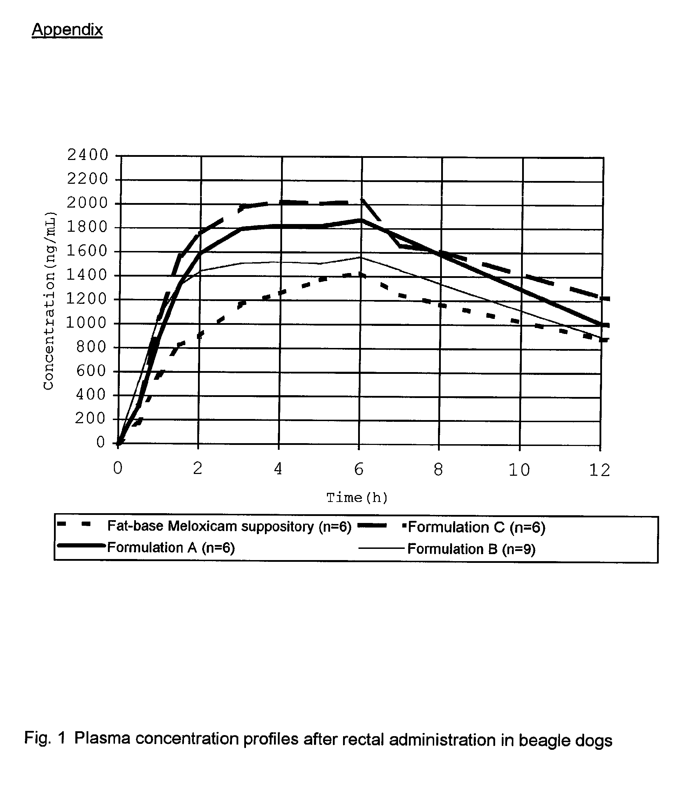

The tested suppositories were prepared from the compositions shown in Table 1. Test results are shown in Fig. 1.

Compositions according to the invention were found to be superior to the fat based composition regarding the onset of meloxicam absorption and the bioavailability of meloxicam.

Stability tests have been conducted with formulation type A at 30 °C, 70 % room humidity over six month in a PVC/aluminium pillow. Details of the stability test can be found in Table 2.

**Table 2.**

| Stability Test | | | |
|---|---|---|---|
| Storage time: 6 [month] | | Sample No. 1 | Sample No. 2 |
| Meloxicam content | | 99.3% | 100.4% |
| impurities | 2-amino-5-methyl thiazole | 0.24% | 0.15% |
| | others | 0.25% | 0.10% |
| | Total | 0.49% | 0.25% |

In both samples the meloxicam content and the packaging material were found to be unchanged.

## Claims

1. A suppository containing a composition, essentially consisting of meloxicam or a pharmaceutically acceptable salt thereof and at least one pharmaceutically acceptable excipient, **characterized in that** at least one of the excipients is a polyalkylenglycol.

2. A suppository capsule according to claim 1, **characterized in that** the suppository is a soft gelatine capsule suitable for rectal administration.

3. A suppository capsule according to claim 1 or 2, **characterized in that** the composition comprises the meloxicam meglumin salt.

4. A suppository capsule according to claim 1 or 2, **characterized in that** the composition comprises the free meloxicam base.

5. A suppository according to claim 1 to 4, **characterized in that** the polyalkylenglycol is selected from the group consisting of polyethylenglycol 400, polyethylenglycol 4000 and polyethylenglycol 6000.

6. A suppository according to claim 1 to 5 **characterized in that** it contains 0.1 % by weight or more of a solubilizer or a mixture of solubilizers of the overall weight of said composition.

7. A suppository according to claim 1 to 6 **characterized in that** the composition contains 1 to 20 µg of the free meloxicam base per mg of the composition.

8. A suppository according to claim 1 to 7 **characterized in that** the composition contains at least 0.2 mg of the solubilizer or mixture of solubilizers per mg of the free meloxicam base.

9. A suppository according to claim 1 to 8 which contains a composition consisting of
a) 1 to 20 mg of meloxicam,
b) 500 to 1500 mg of a one or more polyalkylenglycols selected from the group consisting of polyethylenglycol 400, polyethylenglycol 4000 and polyethylenglycol 6000, and optionally
c) one or two additional excipients.

10. A method for preparation of a suppository according to claim 1 to 9, which comprises the steps of
a) intimately mixing of meloxicam or a pharmaceutically acceptable salt thereof with the melted solubilizer, and
b) encapsulating or molding of the composition obtained in step a) by a conventional method.

11. Use of a suppository according to claim 1 to 9 for the preparation of a medicament with enhanced bioavailability for the treatment or prevention of polyarthritis, rheumatoid arthritis or inflammation deseases.
